# FASCICULE DE BREVET EUROPEEN

(11) **EP 3 209 223 B1**
(45) Date de publication et mention de la délivrance du brevet: **10.10.2018**
(21) Numéro de dépôt: 15807945.9
(22) Date de dépôt: 22.10.2015
(51) Int. Cl.: A61B 17/15

(54) **DISPOSITIF POUR LA PRÉPARATION D'UNE ROTULE POUR LA MISE EN PLACE D'UN IMPLANT ROTULIEN**
VORRICHTUNG ZUR HERSTELLUNG EINER PATELLA ZUR PLATZIERUNG EINES PATELLAIMPLANTATS
DEVICE FOR PREPARING A PATELLA FOR PLACEMENT OF A PATELLAR IMPLANT

(30) Priorité: 24.10.2014 FR 1460248
(43) Date de publication de la demande: 30.08.2017
(73) Titulaire: Aston Medical, 42000 Saint-Etienne (FR)
(72) Inventeur: BOUXIN, Bertrand, F-62180 Verton (FR); CHABERNAUD, Didier, F-61100 Flers (FR)
(74) Mandataire: Cabinet Laurent & Charras
(86) Numéro de dépôt international: PCT/FR2015/052846
(87) Numéro de publication internationale: WO 2016/062973

(56) Documents cités:
- EP-A1- 2 208 470
- US-A- 5 342 364
- US-A- 5 658 291
- US-A1- 2008 097 450

## Description

L'invention se rattache au secteur technique des instruments, notamment pour implants orthopédiques.

Plus particulièrement, l'invention concerne un dispositif de positionnement et de coupe personnalisée d'une rotule, et trouve une application avantageuse dans l'arthroplastie de genou, et plus particulièrement le resurfaçage de la rotule.

De manière parfaitement connu par un homme du métier, une prothèse de genou, et plus particulièrement l'implant fémoral que comprend cette prothèse présente, entre des patins condyliens, une trochlée sur laquelle est susceptible de glisser, lors de la flexion du membre inférieur, la rotule. Afin d'éviter les douleurs, le praticien doit respecter, lors de la mise en place de l'implant prothétique rotulien, l'orientation dudit implant par rapport à la trochlée, afin d'éviter tous phénomènes de ressauts. Il convient également de respecter l'épaisseur, afin d'éviter une tension inappropriée du ligament rotulien sur la trochlée de l'implant prothétique susceptible de générer une limitation de la flexion de genou.

Généralement, la préparation de la rotule anatomique lors de la mise en place d'un implant prothétique rotulien, s'effectue au moyen d'une pince assujettie à un guide de coupe. Toutefois, la conception de cette pince ne permet pas de préparer la rotule aux cas pathologiques spécifiques des patients à prothéser.

US 5 342 364 A décrit un dispositif conforme au préambule de la revendication 1.

L'invention s'est fixée pour but de remédier à ces inconvénients d'une manière simple, sûre, efficace et rationnelle.

Le problème que se propose de résoudre l'invention est de pouvoir préparer la rotule anatomique en adaptant, notamment, le positionnement et la hauteur de coupe pour la mise en place de l'implant rotulien, d'une manière personnalisée à la rotule du patient, notamment à la face postérieure de la rotule à prothéser.

Pour résoudre un tel problème, il a été conçu et mis au point un dispositif pour la préparation d'une rotule pour la mise en place d'un implant rotulien, comprenant, d'une manière connue, une pince assujettie à un guide de coupe et comportant deux branches articulées à l'encontre d'un organe élastique de rappel.

Selon l'invention, le dispositif comprend un corps volumétrique conçu sur mesure et faisant office d'espaceur en étant conformé pour être accouplé à l'une des branches, l'une des faces du corps, considérée à l'opposé de l'accouplement avec la branche, présente une empreinte en creux épousant, en forme inversée, la face antérieure de la rotule positionnée sur l'autre branche, ladite empreinte et la hauteur du corps volumétrique étant définies par un logiciel de modélisation de forme de données scanner ou IRM de ladite rotule, afin de donner l'orientation et le positionnement de la rotule prothétique, la hauteur de coupe postérieure et la profondeur de perçage d'un plot de fixation de l'implant rotulien, le corps volumétrique faisant office d'espaceur est percé coaxialement d'un trou de diamètre sensiblement égal à celui du plot de fixation de l'implant rotulien.

Ces caractéristiques permettent de donner l'orientation et le positionnement de la rotule prothétique, la hauteur de coupe postérieure et la profondeur de perçage du plot de fixation de l'implant prothétique rotulien.

Pour résoudre le problème poser d'accoupler le corps volumétrique à l'une des branches de la pince, notamment la branche supérieure, le trou du corps volumétrique est prolongé par un plot de centrage apte à coopérer avec une forme complémentaire que présente l'une des branches de la pince, notamment la branche supérieure, ledit corps présentant une surface d'appui plane coopérant avec ladite branche.

Pour résoudre le problème posé de déterminer la bonne profondeur de perçage de la rotule anatomique, la branche, où est accouplé le corps volumétrique, fait office de butée à une mèche engagée dans le trou du corps volumétrique. Ladite butée correspond à la profondeur de perçage de la rotule compte tenu de la hauteur adaptée dudit corps.

Dans une forme de réalisation, les deux branches du dispositif reçoivent chacune à leur extrémité libre, un bras articulé, lesdits bras recevant le corps volumétrique et étant montés avec capacité de déplacement en hauteur pour être écartés ou rapprochés l'un de l'autre, d'une manière parallèle, en combinaison avec une colonne de guidage recevant l'organe de rappel.

Pour résoudre le problème posé de positionner la rotule anatomique sur l'une des branches de la pince en vue du positionnement du corps volumétrique pour la préparation de ladite rotule, l'extrémité du bras relatif à la branche inférieure, présente des aspérités d'ancrage coopérant avec la rotule.

Pour résoudre le problème posé de réaliser la découpe de la face antérieure de la rotule, le guide de coupe du dispositif est monté avec capacité de réglage en hauteur sur la colonne de guidage.

L'invention est exposée ci-après plus en détails à l'aide des figures des dessins annexés dans lesquels :

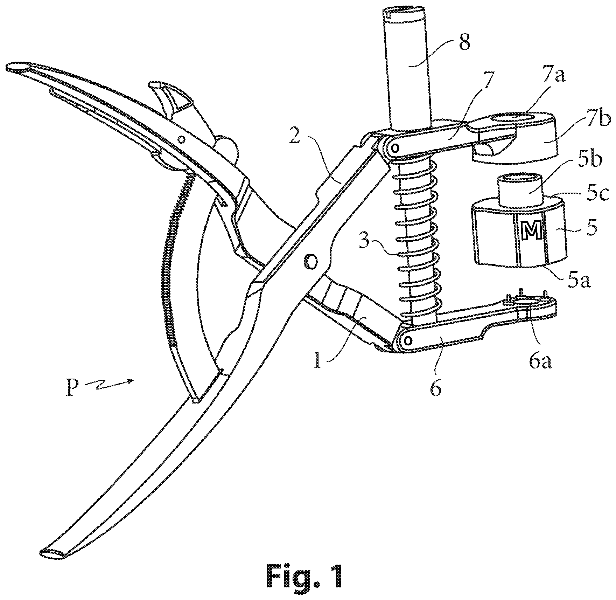

- la figure 1 est une vue en perspective du dispositif, avant mise en place de l'espaceur par rapport à la rotule anatomique,
- la figure 2 montre le dispositif pour la préparation de la rotule positionnée en relation avec l'espaceur, après serrage des branches de la pince,
- la figure 3 montre le dispositif avant perçage de l'empreinte du plot rotulien avec une mèche,
- la figure 4 est une vue en perspective du dispositif, avant mise en place du guide de coupe,
- la figure 5 est une vue en perspective du dispositif, en position serrée des branches avec l'espaceur et la rotule anatomique, le guide de coupe étant bloqué en position basse sur la face de la branche supérieure, et déterminé par la bonne hauteur de l'espaceur afin d'assurer la découpe de la face antérieure de la rotule anatomique, moyennant un organe de coupe, tel qu'une scie.

Le dispositif, selon l'invention, pour l'élaboration d'une rotule, comprend une pince désignée dans son ensemble par (P) et présentant deux branches (1) et (2), articulées à l'encontre d'un organe élastique de rappel (3), notamment sous forme d'un ressort. Cette pince (P) est, par ailleurs, assujettie à un guide de coupe (4).

Selon une caractéristique de l'invention, la pince comprend un corps volumétrique (5), conçu sur mesure, de forme générale parallépipédique, et faisant office d'espaceur. L'espaceur (5) est destiné à être temporairement accouplé à l'une des branches sur la pince, notamment la branche supérieure (2), comme il sera indiqué ultérieurement.

D'une manière importante, l'une des faces (5a) du corps (5), considérée à l'opposé de la face d'accouplement avec la branche (2), présente une empreinte en creux, épousant, en forme inversée, la face antérieure de la rotule anatomique du patient positionnée sur l'autre branche (1). Cette empreinte en creux et la hauteur du corps volumétrique (5) sont définis par un logiciel de de modélisation de forme des données scanner ou IRM de la rotule anatomique. Ces dispositions permettent de donner l'orientation et le positionnement de la rotule prothétique, la hauteur de coupe postérieure, la profondeur de perçage du plot de fixation que présente l'implant rotulien.

Dans la forme de réalisation illustrée, les deux branches (1) et (2) reçoivent chacune à leurs extrémités libres un bras articulé (6) et (7). Les bras (6) et (7) sont montés avec capacité de déplacement en hauteur pour être écartés ou rapprocher l'un de l'autre, de manière parallèle, en combinaison avec une colonne de guidage (8) sous l'effet de l'actionnement des branches (1) et (2) de la pince. La colonne de guidage (8) reçoit le ressort de rappel (3) monté entre les deux bras (6) et (7).

Le corps volumétrique (5) est percé coaxialement d'un trou débouchant dont le diamètre correspond sensiblement à celui du plot de fixation de l'implant prothétique rotulien. Ce trou est prolongé par un plot de centrage (5b) apte à coopérer avec une empreinte complémentaire débouchante (7a) que présente une portée circulaire (7b), formée à l'extrémité libre du bras (7) de la branche supérieure (2). Après engagement du plot (5b) dans l'empreinte (7a) de la portée (7b), une face d'appui plane (5c) de l'espaceur (5), coopère avec ladite portée (7b).

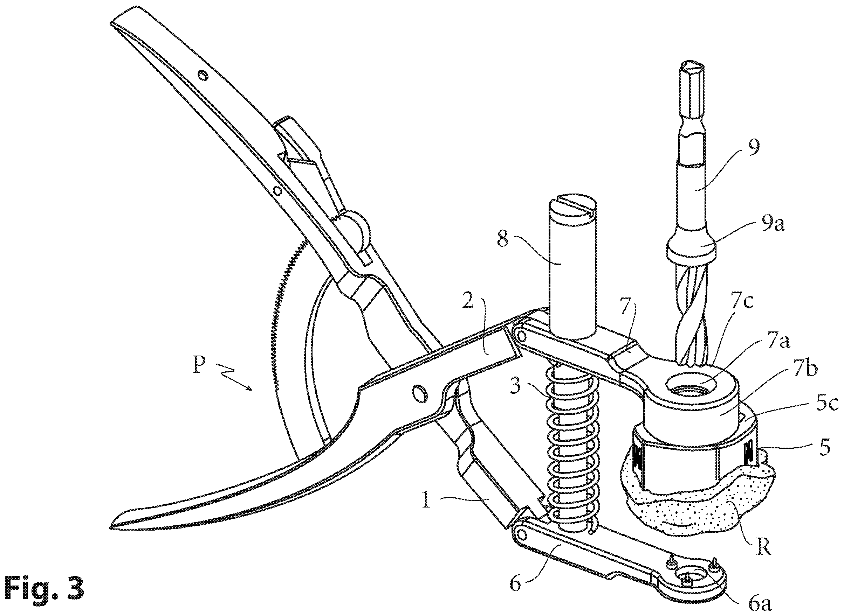

Le bras (7), où est accouplé le corps volumétrique (5), fait office de butée à une mèche (9) engagée dans les trous alignés de l'empreinte (7a) de la portée (7b), du plot (5b) et du corps (5). Dans l'exemple illustré, comme le montre la figure 3, le corps de la mèche (9) présente une butée (9a) apte à prendre appui sur la face supérieure du bras (7) au niveau de la face (7c) de la portée circulaire (7b).

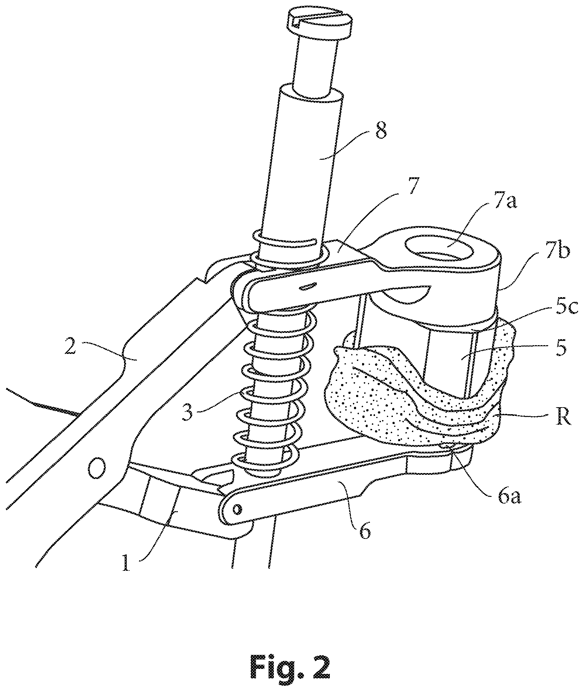

L'extrémité libre du bras inférieur (6) présente des aspérités d'ancrage coopérant avec la rotule anatomique, comme le montre la figure 2.

Il ressort de ces caractéristiques que l'espaceur (5), eu égard à la surface d'appui qui épouse en forme inversée la face intérieure de la rotule anatomique, laquelle forme est définie par un logiciel de modélisation de forme des données scanner ou IRM que le praticien aura demandé à son patient, permet de définir le positionnement et l'orientation de la pince avec la face articulaire postérieure de la rotule.

Comme indiqué, la figure 3 montre la préparation de la rotule (R) positionnée par rapport au corps volumétrique (5), avant en perçage du trou au plot rotulien, avec la mèche (9), positionnée dans l'orifice (7a) et en butée par sa portée (9a) sur la face (7c) du bras (7). Cette butée détermine, grâce à la hauteur adaptée de la forme volumétrique (5), la bonne profondeur de perçage de la rotule anatomique.

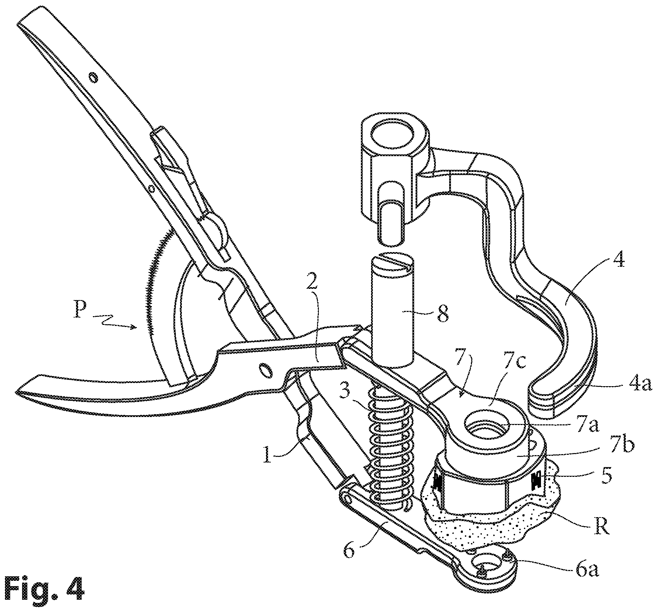

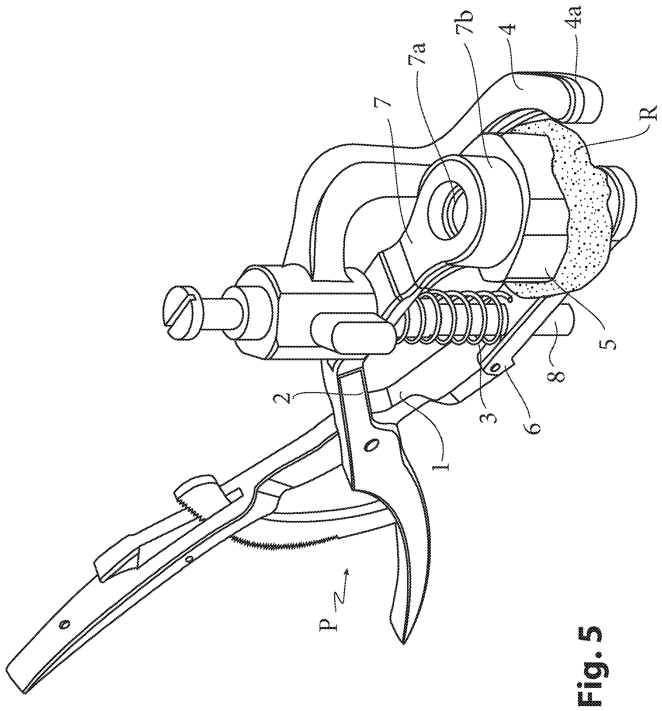

La figure 4 montre la préparation de la rotule anatomique (R) assujettie à l'espaceur (5), avant mise en place du guide de coupe (4) sur la colonne (8) et la figure 5, le bloc de coupe (4) est mis en place sur la colonne (8) et est bloqué en position basse sur la face supérieure du bras (7), par exemple au moyen d'une molette. Le guide de coupe (4) est positionné à la bonne hauteur de coupe en considérant la bonne hauteur du corps volumétrique (5), laquelle hauteur est réalisée sur mesure, suite aux données scanner ou IRM, permettant par conséquent la découpe de la face articulaire postérieure de la rotule anatomique au moyen, par exemple, d'une lame de scie insérée dans une fente (4a), que présente le guide de coupe (4). La rotule prothétique se trouve, par conséquent, dans l'orientation souhaitée.

Les avantages ressortent bien de la description.

## Revendications

1. Dispositif pour la préparation d'une rotule pour la mise en place d'un implant rotulien, comprenant une pince assujettie à un guide de coupe (4) et comportant deux branches articulées (1) et (2) à l'encontre d'un organe élastique de rappel (3), le dispositif comprenant un corps volumétrique (5) faisant office d'espaceur et conformé pour être accouplé à l'une des branches (2), l'une des faces du corps (5) considérée à l'opposé de l'accouplement avec la branche (2) présentant une empreinte en creux, **caractérisé en ce que** le corps volumétrique est conçu sur mesure, son empreint en creux épousant en forme inversée, la face articulaire postérieure de la rotule positionnée sur l'autre branche (1), ladite empreinte et la hauteur du corps volumétrique (5) étant définies par un logiciel de modélisation de forme de données scanner ou IRM de ladite rotule, afin de donner l'orientation et le positionnement de la rotule prothétique, la hauteur de coupe postérieure et la profondeur de perçage d'un plot de fixation de l'implant rotulien, le corps volumétrique (5) faisant office d'espaceur, étant percé coaxialement d'un trou débouchant de diamètre sensiblement égal à celui du plot de fixation de l'implant rotulien.

2. Dispositif selon la revendication 1, ***caractérisé* en ce que** le trou du corps volumétrique (5) est prolongé par un plot de centrage (5b) apte à coopérer avec une forme complémentaire que présente l'une des branches (2) de la pince notamment la branche supérieure, ledit corps (5) présentant une surface d'appui plane (5c) coopérant avec ladite branche (2).

3. Dispositif selon la revendication 2, ***caractérisé* en ce que** la branche (2) où est accouplé le corps volumétrique (5), fait office de butée à une mèche (9) engagée dans le trou du corps volumétrique (5), ladite butée correspond à la profondeur de perçage de la rotule compte tenu de la hauteur adaptée dudit corps.

4. Dispositif selon la revendication 1, ***caractérisé* en ce que** les deux branches (1) et (2) reçoivent chacune à leur extrémité libre, un bras articulé (6) et (7), lesdits bras recevant le corps volumétrique (5) et étant montés avec capacité de déplacement en hauteur pour être écartés ou rapprochés l'un de l'autre, d'une manière parallèle, en combinaison avec une colonne de guidage (8) recevant l'organe de rappel (3).

5. Dispositif selon l'une quelconque des revendications 1 à 4, ***caractérisé* en ce que** l'extrémité du bras (6) relatif à la branche inférieure (1), présente des aspérités d'ancrage coopérant avec la rotule.

6. Dispositif selon les revendications 1 et 4, ***caractérisé* en ce que** le guide de coupe (4) est monté avec capacité de réglage en hauteur sur la colonne de guidage.

## Patentansprüche

1. Vorrichtung zur Herstellung eines Kugelgelenkes zum Einsetzen eines Patella-Implantats, mit einer Zange, kombiniert mit einer Schneidführung (4) und mit zwei angelenkten Schenkel (1) und (2) gegenüber einer elastischen Rückstellvorrichtung (3), wobei die Vorrichtung einen Verdrängerkörper (5) umfasst, der als Abstandshalter dient und so gestaltet ist, dass er mit einem der Schenkel (2) verbunden werden kann, eine der Seiten des Verdängerkörpers (5), vorgesehen gegenüber der Verbindung mit dem Schenkel (2) weist eine hohle Aussparung auf, **dadurch gekennzeichnet, dass** der Verdrängerkörper nach Maß konzipiert ist, seine hohle Aussparung schmiegt sich in umgekehrter Form der hinteren, angelenkten Seite der Patella an, die sich auf dem anderen Schenkel (1) befindet, diese Aussparung und die Erhöhung des Verdrängerkörpers (5) werden durch eine Formmodellierungs- Software von Scanner- oder MRT- Daten dieser Patella definiert, um die Ausrichtung und Positionierung der Patella-Prothese, die Höhe des posterioren Schnitts und die Eindringtiefe eines Befestigungsstiftes des Patella- Implantats anzugeben, der Verdrängerkörper (5) dient dabei als Abstandshalter und durch ihn führt koaxial ein einmündendes Loch hindurch, dessen Durchmesser im Wesentlichen dem des Befestigungsstiftes des Patella-Implantats entspricht.

2. Vorrichtung gemäß Anspruch 1, **dadurch gekennzeichnet, dass** das Loch des Verdrängerkörpers (5) sich durch einen Zentrierstift (5b) verlängert, der in der Lage ist, mit einer komplementären Form zusammenzuwirken, die einer der Schenkel (2) der Zange aufweist, insbesondere der obere Schenkel, wobei dieser Körper (5) eine flache Auflagefläche (5c) aufweist, die mit diesem Schenkel (2) kooperiert.

3. Vorrichtung gemäß Anspruch 2, **dadurch gekennzeichnet, dass** der Schenkel (2) mit dem der Verdrängerkörper (5) verbunden ist, als Anschlag einer Bohrspitze (9) dient, die in das Loch des Verdrängerkörpers (5) eingeführt wird, dieser Anschlag entspricht der Tiefe der Patella- Bohrung unter Berücksichtigung der passenden Höhe dieses Körpers.

4. Vorrichtung gemäß Anspruch 1, **dadurch gekennzeichnet, dass** die beiden Schenkel (1) und (2) jeweils an ihren freien Endstück mit einem angelenkten Arm (6) und (7) versehen werden, diese Arme nehmen den Verdrängerkörper (5) auf und sind in der Höhe verschiebbar montiert, so dass sie voneinander entfernt oder einander angenähert werden können, parallel, in Kombination mit einer Führungssäule (8), die die Rückstellvorrichtung (3) aufnimmt.

5. Vorrichtung gemäß irgendeinem der Ansprüche 1 bis 4, **dadurch gekennzeichnet, dass** das Endstück des Arms (6) bezogen auf den unteren Schenkel (1) Verankerungsunebenheiten aufweist, die mit der Patella kooperieren.

6. Vorrichtung gemäß den Ansprüchen 1 bis 4, **dadurch gekennzeichnet, dass** die Schneidführung (4) mit einer Höhenverstellung auf der Führungssäule montiert ist.

## Claims

1. Device for the preparation of a patella for the implementation of a patellar implant, comprising a clamp secured to a cutting guide (4) and comprising 2 arms (1) and (2) articulated against an elastic return means (3), the device comprising a volumetric body (5) acting as a spacer, and being shaped such as to be coupled to one of the arms (2), one of the faces of the body (5), considered to be opposite to the coupling with the arm (2) having a recessed indentation **characterized in that** the volumetric body is custom-designed, its recessed indentation that is married, in inverted form, with the posterior articular face of the patella positioned on the other arm (1), said indentation and the height of the volumetric body (5) being defined by means of form modeling software using scanner or MRI data from said patella, in order to give the orientation and positioning of the prosthetic patella, the posterior cutting height and the drilling depth of a retention pin for the patellar implant, the volumetric body (5) acting as a spacer being coaxially drilled with a through hole of a diameter that is substantially equal to that of the patellar implant retention pin.

2. Device according to claim 1, ***characterized* in that** the hole of the volumetric body (5) is extended by means of a centering pin (5b) that is capable of cooperating with a complementary shape of one of the arms (2) of the clamp, in particular the upper arm, said body (5) having a planar support surface (5c) cooperating with said arm (2).

3. Device according to claim 2, ***characterized* in that** the arm (2) whereupon the volumetric body (5) is coupled, serves as an abutment for a drill (9) engaged in the hole of the volumetric body (5), said abutment being located at the drilling depth of the patellar taking into consideration the adapted height of said body.

4. Device according to claim 1, ***characterized* in that** the two arms (1) and (2) each receive at their free end, an articulated arm (6) and (7), said arms receiving the volumetric body (5) and being mounted with height-displacement capability in order to be spaced apart or brought closer together, in a parallel manner, in combination with a guide column (8) receiving the return means (3).

5. Device according to any of claims 1 to 4, ***characterized* in that** the end of the arm (6) relative to the lower arm (1), has rough anchoring patches that cooperate with the patella.

6. Device according to claims 1 and 4, in that the cutting guide (4) is mounted with height adjustment on the guide column.
